# EUROPÄISCHE PATENTANMELDUNG

(11) **EP 4 000 642 A1**
(43) Veröffentlichungstag der Anmeldung: **25.05.2022**
(21) Anmeldenummer: 20207785.5
(22) Anmeldetag: 16.11.2020
(51) Int. Cl.: A61L 2/07, A61L 2/08, B01D 65/02, A61L 2/26, B01D 15/20

(54) **VERFAHREN ZUR SICHERSTELLUNG EINER MIKROBIOLOGISCHEN REINHEIT EINER EINWEG-VORRICHTUNG SOWIE DECKEL FÜR EINEN FLUIDANSCHLUSS EINER SEPARATIONSEINHEIT ZUR VERWENDUNG IN EINEM SOLCHEN VERFAHREN**

(71) Anmelder: Sartorius Stedim Biotech GmbH, 37079 Göttingen (DE)
(72) Erfinder: WORTMEYER, Johannes, 37079 Göttingen (DE); LOEWE, Thomas, 37079 Göttingen (DE); MIETH, Florian, 37079 Göttingen (DE)
(74) Vertreter: Prinz & Partner mbB

(57) **Zusammenfassung**

Bei einem Verfahren zur Sicherstellung einer mikrobiologischen Reinheit einer Einweg-Vorrichtung (10) zur Durchführung eines bioverfahrenstechnischen Prozesses umfasst die Einweg-Vorrichtung (10) wenigstens eine gammasterilisierbare Komponente, die aus für eine Sterilisierung durch Gammastrahlung geeigneten Materialien gebildet ist, und wenigstens eine nicht-gammasterilisierbare Untereinheit, die ein für eine Sterilisierung durch Gammastrahlung ungeeignetes Material enthält. Sowohl die Komponente als auch die Untereinheit weisen jeweils einen Bereich auf, der bei der Durchführung des bioverfahrenstechnischen Prozesses mit einem Prozess-Medium in Berührung kommt. Das Verfahren umfasst folgende Schritte: a) Sterilisieren der gammasterilisierbaren Komponente durch Gammastrahlung; b) Schützen des Medium-berührenden Bereichs der gammasterilisierbaren Komponente durch eine Sterilbarriere; c) Sterilisieren der nicht-gammasterilisierbaren Untereinheit mit Heißdampf; d) Schützen des Medium-berührenden Bereichs der nicht-gammasterilisierbaren Untereinheit durch eine Sterilbarriere; e) Entfernen der Sterilbarrieren; und f) Montieren der gammasterilisierten Komponente und der Heißdampf-sterilisierten Untereinheit in der Einweg-Vorrichtung (10) unmittelbar nach dem Entfernen der Sterilbarrieren.

## Beschreibung

Die Erfindung betrifft ein Verfahren zur Sicherstellung einer mikrobiologischen Reinheit einer Einweg-Vorrichtung zur Durchführung eines bioverfahrenstechnischen Prozesses, insbesondere einer Einweg-Filtrationsvorrichtung. Die Erfindung betrifft ferner einen Deckel für einen Fluidanschluss einer Separationseinheit. Außerdem betrifft die Erfindung eine Verwendung eines Deckels für einen Fluidanschluss einer Separationseinheit in einem Verfahren zur Sicherstellung einer mikrobiologischen Reinheit einer Einweg-Vorrichtung.

Eine Einweg-Vorrichtung im Sinne der Erfindung ist eine zum einmaligen Gebrauch vorgesehene Vorrichtung, die anschließend nicht für eine erneute Verwendung gereinigt und sterilisiert, sondern in der Regel vollständig entsorgt wird. Dementsprechend unterscheidet sich eine Einweg-Vorrichtung hinsichtlich der für ihre einzelnen Komponenten verwendeten Materialien (vorwiegend Kunststoffe) von einer wiederverwendbaren Vorrichtung, die z. B. Komponenten aus Edelstahl aufweist.

Unter einer Separationseinheit ist im Zusammenhang der vorliegenden Erfindung z. B. eine Filtercapsule, eine Chromatographiesäule oder ein Membranadsorber zu verstehen. Grundsätzlich ist die Erfindung aber auch bei Einweg-Vorrichtungen mit anderen Funktionseinheiten anwendbar, insbesondere wenn diese - im Gegensatz zu anderen Komponenten der Einweg-Vorrichtung - nicht für eine Sterilisierung durch Gammastrahlung geeignet sind. Hierauf wird später noch eingegangen.

Grundsätzlich besteht der Wunsch eines Anwenders einer Einweg-Vorrichtung zur Durchführung eines bioverfahrenstechnischen Prozesses, insbesondere einer Einweg-Filtrationsvorrichtung, dass die Einweg-Vorrichtung nach der Auslieferung sofort einsatzbereit ist. Voraussetzung hierfür ist in der Regel, dass die Einweg-Vorrichtung bestimmten Anforderungen an die mikrobiologische Reinheit genügt. In vielen Anwendungsfällen ist diese unverzichtbar, denn ansonsten besteht die Gefahr, dass die Funktions- bzw. Leistungsfähigkeit der Separationseinheiten nicht gewährleistet und/oder dass das Zielprodukt verunreinigt ist. Letzteres ist besonders prekär, wenn die Verunreinigung dem Zielprodukt ähnlich ist und somit auch nachträglich nicht effektiv herausgefiltert werden kann.

Für den Anwender bedeutet es jedoch eine zusätzliche Belastung, wenn er selbst für die gewünschte mikrobiologische Reinheit der Einweg-Vorrichtung sorgen muss. Es besteht daher der Wunsch, dass die Einweg-Vorrichtung bereits auf Seiten des Herstellers sterilisiert wird. Damit die Einweg-Vorrichtung in sterilem Zustand an den Anwender ausgeliefert werden kann, muss sie mit einer Sterilbarriere versehen werden, die gleichzeitig als Verpackung für den Transport dienen kann.

Die Sterilisierung einer gesamten Einweg-Vorrichtung kann jedoch aus verschiedenen Gründen problematisch sein. Wenn eine Einweg-Vorrichtung Separationseinheiten oder andere Funktionseinheiten enthält, die nicht auf die gleiche Weise sterilisiert werden können wie die anderen Medium-berührenden Komponenten der Einweg-Vorrichtung, sind getrennte Sterilisierungsvorgänge für die jeweiligen Komponenten erforderlich. Das bedeutet, dass die Einweg-Vorrichtung nicht nach dem Zusammenbau als Ganzes sterilisiert werden kann.

Grundsätzlich ist eine Sterilisierung durch Gammastrahlung vorteilhaft, da sie die Abmessungen der Komponenten nicht beeinträchtigt (Maßhaltigkeit). Gewisse Materialien, die bei Separationseinheiten z. B. als Filter- oder Membranmaterial verwendet werden, wie etwa Polytetrafluorethylen (PTFE), Polypropylen (PP) oder Polyvinylchlorid (PVC), sind jedoch für eine Sterilisierung durch Gammastrahlung nicht geeignet, da sie infolge der Bestrahlung wichtige Materialeigenschaften verlieren, insbesondere ihre mechanische Stabilität.

Separationseinheiten mit solchen Materialien müssen deshalb auf andere Weise sterilisiert werden, vorzugsweise durch Heißdampf in einem Autoklav. Die Heißdampfsterilisierung hat jedoch den Nachteil, dass der Überdruck im Autoklav zu einer unerwünschten plastischen Verformung der Separationseinheiten führen kann.

Wenn die Einweg-Vorrichtung als Ganzes ausgeliefert werden soll, besteht außerdem das Problem, dass vor, während und nach dem Zusammenbau der in getrennten Prozessen sterilisierten Separationseinheiten und übrigen Komponenten Verunreinigungen in das Innere der Separationseinheiten und der anderen Komponenten gelangen können.

Vor diesem Hintergrund ist es die Aufgabe der Erfindung, die Sicherstellung einer mikrobiologischen Reinheit einer Einweg-Vorrichtung zur Durchführung eines bioverfahrenstechnischen Prozesses, insbesondere einer Einweg-Filtrationsvorrichtung, vor deren Inbetriebnahme zu verbessern und zu vereinfachen.

Gelöst wird diese Aufgabe durch ein Verfahren mit den Merkmalen des Anspruchs 1 und durch ein Verfahren mit den Merkmalen des Anspruchs 3. Vorteilhafte und zweckmäßige Ausgestaltungen des erfindungsgemäßen Verfahrens sind in den zugehörigen Unteransprüchen angegeben.

Das erfindungsgemäße Verfahren zur Sicherstellung einer mikrobiologischen Reinheit ist für eine Einweg-Vorrichtung zur Durchführung eines bioverfahrenstechnischen Prozesses vorgesehen, wobei die Einweg-Vorrichtung wenigstens eine gammasterilisierbare Komponente, die aus für eine Sterilisierung durch Gammastrahlung geeigneten Materialien gebildet ist, und wenigstens eine nicht-gammasterilisierbare Untereinheit umfasst, die ein für eine Sterilisierung durch Gammastrahlung ungeeignetes Material enthält. Sowohl die Komponente als auch die Untereinheit weisen jeweils einen Bereich auf, der bei der Durchführung des bioverfahrenstechnischen Prozesses mit einem Prozess-Medium in Berührung kommt.

Gemäß einem ersten Aspekt umfasst das erfindungsgemäße Verfahren folgende Schritte: a) Sterilisieren der gammasterilisierbaren Komponente durch Gammastrahlung; b) Schützen des Medium-berührenden Bereichs der gammasterilisierbaren Komponente durch eine Sterilbarriere; c) Sterilisieren der nicht-gammasterilisierbaren Untereinheit mit Heißdampf; d) Schützen des Medium-berührenden Bereichs der nicht-gammasterilisierbaren Untereinheit durch eine Sterilbarriere; e) Entfernen der Sterilbarrieren; und f) Montieren der gammasterilisierten Komponente und der Heißdampf-sterilisierten Untereinheit in der Einweg-Vorrichtung unmittelbar nach dem Entfernen der Sterilbarrieren.

Die Erfindung beruht auf der Erkenntnis, dass bei einer Einweg-Vorrichtung, insbesondere einer Einweg-Vorrichtung mit einer oder mehreren Separationseinheiten, die nicht nach dem Zusammenbau als Ganzes sterilisiert werden kann, die Gefahr einer Verunreinigung in der Phase vor und während des Zusammenbaus der Einweg-Vorrichtung am größten ist. Die Erfindung sieht deshalb besondere Schutzmaßnahmen vor:
Die gammasterilisierbare Komponente und die nicht-gammasterilisierbare Untereinheit werden in getrennten Vorgängen sterilisiert, und die Medium-berührenden Bereiche der gammasterilisierbaren Komponente und der nicht-gammasterilisierbaren Untereinheit werden jeweils durch eine Sterilbarriere geschützt. Eine solche Sterilbarriere kann insbesondere eine Hülle oder eine Verpackung für die gesamte Komponente bzw. Untereinheit sein, oder ein Verschluss, wie etwa eine Blindkappe oder ein Sterilkonnektor, der den jeweiligen Medium-berührenden Bereich schützt, wie später noch genauer erläutert wird.

Erst unmittelbar vor dem Montieren der gammasterilisierten Komponente und der Heißdampf-sterilisierten Untereinheit werden die Sterilbarrieren entfernt.

Sofern nach dem Entfernen der Sterilbarrieren keine weiteren Schutzmaßnahmen vorgesehen sind, wie etwa zusätzliche Verschlüsse, auf die später noch eingegangen wird, sollte der Zeitraum zwischen dem Entfernen einer Sterilbarriere und dem Montieren der zugehörigen gammasterilisierten Komponente bzw. der zugehörigen Heißdampf-sterilisierten Untereinheit je nach Partikelkonzentration in der Umgebung kürzer als zwei Minuten, vorzugsweise kürzer als eine Minute und weiter vorzugsweise kürzer als eine halbe Minute sein. Praxistests haben ergeben, dass bei Einhaltung dieser Zeiträume die Gefahr einer Verunreinigung der Medium-berührenden Bereiche der Einweg-Vorrichtung sehr effektiv gesenkt wird.

Grundsätzlich können die wenigstens eine nicht-gammasterilisierbare Untereinheit und die wenigstens eine gammasterilisierbare Komponente zu unterschiedlichen Zeiten sterilisiert werden, ohne dass eine Reihenfolge zu beachten ist.

Gemäß einem zweiten Aspekt der Erfindung kommt zusätzlich zu den Sterilbarrieren (unter Umständen kann auch eine gemeinsame Sterilbarriere sowohl für die wenigstens eine gammasterilisierbare Komponente als auch für die wenigstens eine nicht-gammasterilisierbare Untereinheit vorgesehen sein) wenigstens ein Verschluss zum Einsatz, der den Medium-berührenden Bereich der gammasterilisierbaren Komponente bzw. der nicht-gammasterilisierbaren Komponente abdeckt. Ein solcher, von der Sterilbarriere unabhängiger Verschluss muss keine Sterilbarriere bilden, solange er zumindest dazu beiträgt, dass die Wahrscheinlichkeit eines unerwünschten Partikeleintrags signifikant gesenkt wird. Nach dem Entfernen der Sterilbarriere verbleibt dann dank des zusätzlichen Verschlusses ein erweitertes Zeitfenster für die Montage der betreffenden Komponente bzw. Untereinheit.

Gemäß diesem zweiten Aspekt umfasst das erfindungsgemäße Verfahren folgende Schritte: a) Sterilisieren der gammasterilisierbaren Komponente durch Gammastrahlung; b) Schützen des Medium-berührenden Bereichs der gammasterilisierbaren Komponente durch eine Sterilbarriere; c) Sterilisieren der nicht-gammasterilisierbaren Untereinheit mit Heißdampf; d) Schützen des Medium-berührenden Bereichs der nicht-gammasterilisierbaren Untereinheit durch eine Sterilbarriere; e) zusätzliches Abdecken des Medium-berührenden Bereichs der gammasterilisierbaren Komponente mit wenigstens einem ersten Verschluss, und/ oder zusätzliches Abdecken des Medium-berührenden Bereichs der nicht-gammasterilisierbaren Untereinheit mit wenigstens einem zweiten Verschluss; f) Entfernen der Sterilbarrieren und späteres Entfernen des ersten und/oder zweiten Verschlusses; und g) Montieren der gammasterilisierten Komponente bzw. der Heißdampf-sterilisierten Untereinheit in der Einweg-Vorrichtung unmittelbar nach dem Entfernen des ersten bzw. zweiten Verschlusses.

Die Bezeichnungen "erster" und "zweiter" Verschluss dienen hier lediglich zur Unterscheidung eines Verschlusses, der für die gammasterilisierbare Komponente vorgesehen ist, von einem Verschluss, der für die nicht-gammasterilisierbaren Untereinheit vorgesehen ist. Ein "erster" Verschluss setzt also nicht zwingend einen "zweiten" Verschluss voraus und umgekehrt.

Konkret kann der erweiterte Zeitraum zwischen dem Entfernen der jeweiligen Sterilbarriere und dem Montieren der zugehörigen, mit einem ersten Verschluss versehenen gammasterilisierten Komponente bzw. der zugehörigen. mit einem zweiten Verschluss versehenen Heißdampf-sterilisierten Untereinheit jeweils kürzer als drei Stunden, vorzugsweise kürzer als zwei Stunden, weiter vorzugsweise kürzer als eine Stunde und weiter vorzugsweise kürzer als eine halbe Stunde sein.

Der Zeitraum zwischen dem Entfernen des ersten bzw. zweiten Verschlusses und dem Montieren der zugehörigen gammasterilisierten Komponente bzw. Heißdampf-sterilisierten Untereinheit sollte aber wiederum jeweils kürzer als zwei Minuten, vorzugsweise kürzer als eine Minute und weiter vorzugsweise kürzer als eine halbe Minute sein.

Sofern zusätzlich zur Sterilbarriere zusätzlich wenigstens ein Verschluss zum Abdecken des Medium-berührenden Bereichs der gammasterilisierbaren Komponente bzw. der nicht-gammasterilisierbaren Komponente vorgesehen ist, sollte ein solcher Verschluss für einen effektiven Schutz eine Partikeleintrag-reduzierende Barriere mit einem Spaltmaß von höchstens 100 µm, vorzugsweise von höchstens 50 µm und weiter vorzugsweise von höchstens 10 µm bilden. Grundsätzlich kann auch ein Verschluss verwendet werden, der selbst eine Sterilbarriere darstellt.

Beim erfindungsgemäßen Verfahren kann des Weiteren vorgesehen sein, dass der Schritt b) vor dem Schritt a) und/oder der Schritt d) vor dem Schritt c) erfolgt, d. h. die Medium-berührende Bereiche der gammasterilisierbaren Komponente bzw. der nicht-gammasterilisierbaren Untereinheit können schon vor dem Sterilisieren mit einer Sterilbarriere geschützt werden. Bei der gammasterilisierbaren Komponente ist dementsprechend ein Gammastrahlen-resistentes Material für die Sterilbarriere zu wählen. Bei der mit Heißdampf sterilisierten Untereinheit ist sicherzustellen, dass der Heißdampf in ausreichendem Maße durch die Sterilbarriere hindurchdringen kann. In beiden Fällen ist dann sichergestellt, dass nach dem Verbringen der sterilisierten Komponente bzw. Untereinheit in eine nichtsterile Umgebung die Medium-berührenden Bereiche jeweils steril bleiben.

Als Sterilbarriere für die Untereinheit, die mit Heißdampf sterilisiert wird, eignet sich grundsätzlich eine die Untereinheit vollständig umgebende, geschlossene Hülle, die aus einem Vliesstoff, vorzugsweise Tyvek^{®}, gebildet ist. Dieser Vliesstoff besteht aus Polyethylen hoher Dichte (PE-HD) und baut sich aus fibrillierten, eng miteinander zu Netzwerken verbundenen Feinstfilamenten im Durchmesserbereich von 0,5 bis 10 µm auf. Ein solches Material zeichnet sich durch seine hohe Wasserdampfdurchlässigkeit aus und erlaubt somit ein schnelles Eindringen von Heißdampf, welcher für die Sterilisierung der in der geschlossenen Hülle befindlichen Untereinheit erforderlich ist. Andererseits eignet sich das Material unter normalen Umgebungsbedingungen als Sterilbarriere. Das Material verliert nur sehr wenige Fasern, und es ist hydrophob, d. h. es zieht kein Wasser in das Material ein. Deshalb behält das Material seine Festigkeit, unabhängig davon, ob es mit Wasser benetzt oder trocken ist. In einer aus einem solchen Material gebildeten Hülle kann die Untereinheit also mit Heißdampf sterilisiert und anschließend gelagert und/oder transportiert werden.

Gemäß einem besonderen Aspekt der Erfindung wird der Medium-berührende Bereich der Untereinheit vor dem Sterilisieren mit Heißdampf und bis zum Montieren der Heißdampf-sterilisierten Untereinheit durch einen zweiten Verschluss, insbesondere einen Deckel, abgedeckt. Der Verschluss weist wenigstens eine Durchgangsöffnung auf, die selektiv freigegeben und verschlossen werden kann. Wie eingangs bereits erwähnt, kann dieser Verschluss die erfindungsgemäß vorgesehene Sterilbarriere für den Medium-berührenden Teil der Untereinheit bilden. Dies ist aber nicht zwingend notwendig, wenn die Sterilbarriere anderweitig, insbesondere durch die zuvor genannte Hülle gebildet ist.

Im Hinblick auf die bevorzugte Anwendung der Erfindung weist die nicht-gammasterilisierbare Untereinheit wenigstens eine Funktionseinheit, insbesondere eine Separationseinheit (z. B. eine Filtercapsule, eine Chromatographiesäule oder einen Membranadsorber), mit wenigstens einem Fluidanschluss auf. Bei der Durchführung des erfindungsgemäßen Verfahrens wird der Fluidanschluss vorzugsweise durch einen zweiten Verschluss abgedeckt. Wenn zusätzlich eine Sterilbarriere vorgesehen ist, wie die zuvor genannte Hülle, kann nach deren Entfernung die Exposition des Fluidanschlusses verkürzt werden, indem der Verschluss erst unmittelbar vor der Montage der Funktionseinheit abgenommen wird. Das bedeutet, dass die Zeitspanne, die für eine wahrscheinlich "reine" (nicht verunreinigte) Montage zur Verfügung steht, verlängert wird, da nach dem Entfernen der Sterilbarriere nicht innerhalb von zwei Minuten alle Fluidanschlüsse montiert werden müssen. Dies ist insbesondere bei komplizierten Aufbauten mit vielen Anschlüssen von Vorteil, da im Zuge der Montage ein Verschluss nach dem anderen entfernt werden kann.

In einer besonders bevorzugten Ausführungsform umfasst das erfindungsgemäße Verfahren folgende weitere Schritte bzw. Maßnahmen: Anbringen des zweiten Verschlusses auf dem Fluidanschluss der Funktionseinheit; Freigeben der Durchgangsöffnung, falls die Durchgangsöffnung verschlossen ist; Verpacken der nicht-gammasterilisierbaren Untereinheit in die Hülle; Sterilisieren der verpackten Untereinheit mit Heißdampf bei freigegebener Durchgangsöffnung; nach dem Sterilisieren mit Heißdampf: Verschließen der Durchgangsöffnung in der geschlossenen Hülle; Auspacken der Heißdampf-sterilisierten Untereinheit aus der geschlossenen Hülle; und Abnehmen des zweiten Verschlusses unmittelbar vor dem Montieren der Untereinheit.

Das bedeutet, dass die Untereinheit mit der Funktionseinheit, insbesondere einer Separationseinheit, zuerst in einer Hülle verpackt und dann in der geschlossenen Hülle sterilisiert wird, sodass nach dem Sterilisieren keine Verunreinigung mehr in die Hülle gelangen kann. Dies ist möglich, da die Hülle - wie zuvor erläutert - aus einem Material hergestellt werden kann, das durchlässig für Heißdampf ist, unter normalen Bedingungen aber als Sterilbarriere wirkt.

Eine weitere Voraussetzung ist, dass sich die Funktionseinheit durch die Heißdampfsterilisierung nicht plastisch verformt. Dies wird dadurch erreicht, dass die Durchgangsöffnung des vor der Sterilisierung angebrachten zweiten Verschlusses während der Heißdampfsterilisierung freigegeben ist, sodass durch den offenen Fluidanschluss der Separationseinheit ein Druckausgleich möglich ist.

Außerdem ist sichergestellt, dass nach dem Auspacken der Untereinheit aus der Hülle weiterhin eine Partikeleintrag-reduzierende Barriere besteht, die den Medium-berührenden Bereich vor Verunreinigungen schützt. Diese Barriere wird durch den zweiten Verschluss bereitgestellt, dessen Durchgangsöffnung nach dem Sterilisieren in der geschlossenen Hülle verschlossen wurde. Somit besteht nach dem Auspacken der Heißdampf-sterilisierten Untereinheit noch solange ein Schutz, bis der zweite Verschluss im Verlauf des Zusammenbaus der Einweg-Vorrichtung abgenommen wird.

Wie bereits erläutert wird gemäß der Erfindung die wenigstens eine gammasterilisierbare Komponente der Einweg-Vorrichtung separat, d. h. nicht zusammen mit der nicht-gammasterilisierbaren Untereinheit sterilisiert, da die Sterilisierung mit Heißdampf das Risiko birgt, dass danach die Maßhaltigkeit der Komponente nicht mehr gegeben ist. Gerade bei einem in dieser Hinsicht kritischem Bauteil, wie etwa einem starren Verbindungsrohr, an dessen Fluidanschlüsse mehrere in einer starren Halterung fixierte Separationseinheiten und weitere Verbindungsrohre angeschlossen werden sollen, ist die Maßhaltigkeit jedoch von essentieller Bedeutung. Diese wird bei der für die Komponente erfindungsgemäß vorgesehene Sterilisierung durch Gammastrahlung nicht beeinträchtigt.

Um bei der gammasterilisierbaren Komponente der Einweg-Vorrichtung auch nach dem Entfernen der erfindungsgemäß vorgesehenen Sterilbarriere die Gefahr einer Verunreinigung des Medium-berührenden Bereichs der Komponente zu minimieren, ist im Einklang mit dem zweiten Aspekt der Erfindung vorgesehen, dass der Medium-berührende Bereich der Komponente durch einen ersten Verschluss, vorzugsweise durch eine Blindkappe oder einen Sterilkonnektor, abgedeckt bleibt. Da die Gammastrahlung die Maßhaltigkeit der Komponente und des Verschlusses, sofern dieser aus einem geeigneten Material gebildet ist, weder kurz- noch langfristig beeinträchtigt (keine elastische oder plastische Verformung, keine Brüchigkeit o.ä.), kann der Verschluss bereits vor dem Sterilisieren angebracht werden und bis zum Montieren der Komponente dort verbleiben. Somit besteht für den Medium-berührenden Bereich der gammasterilisierten Komponente solange ein Schutz, bis der erste Verschluss im Zuge der Montage der Komponente abgenommen wird. Der erste Verschluss zum Abdecken des Medium-berührenden Bereichs der gammasterilisierbaren Komponente kann aber im Einklang mit dem ersten Aspekt der Erfindung auch die alleinige Sterilbarriere für die Komponente bilden.

Im Hinblick auf die die bevorzugte Anwendung der Erfindung umfasst die wenigstens eine gammasterilisierbare Komponente typischerweise ein Verbindungsrohr und/oder eine Mehrzahl von untereinander verbindbaren Verteilerbaugruppen zum Verbinden mehrerer Separationseinheiten und/oder wenigstens eine Anschlussschlauchleitung, wobei die gammasterilisierbare Komponente wenigstens einen Fluidanschluss aufweist, der wie zuvor beschrieben durch den ersten Verschluss abgedeckt wird.

Wenn kein erster Verschluss für die gammasterilisierbare Komponente vorgesehen ist oder ein solcher Verschluss keine Sterilbarriere bildet, ist die erfindungsgemäß vorgesehene Sterilbarriere für den Medium-berührenden Teil der Komponente vorzugsweise durch eine die Komponente vollständig umgebende, geschlossene Primärverpackung gebildet.

Um das Risiko einer Verunreinigung aus der Umgebung weiter zu minimieren, sollten die Heißdampf-sterilisierte Untereinheit und die gammasterilisierte Komponente auf einer Sicherheitswerkbank (Laminar Flow Werkbank, Cleanbench) oder in einem Reinraum, vorzugsweise in einem durch Wände abgetrennten Bereich eines Reinraums, montiert werden.

Die Erfindung schafft auch einen Deckel für einen Fluidanschluss einer nicht-gammasterilisierbaren Untereinheit zur Verwendung in einem erfindungsgemäßen Verfahren, bei dem ein zweiter Verschluss für eine Separationseinheit der Untereinheit vorgesehen ist. Der erfindungsgemäße Deckel umfasst einen Grundkörper zum passgenauen Anbringen des Deckels am Fluidanschluss. Der Deckel hat wenigstens eine Durchgangsöffnung, die nach dem Anbringen des Deckels am Fluidanschluss eine Strömungsverbindung zwischen dem Medium-berührenden Bereich der Separationseinheit und der unmittelbaren Umgebung der Separationseinheit bereitstellt. Der Deckel umfasst ferner einen Mechanismus zum selektiven Freigeben und Verschließen der wenigstens einen Durchgangsöffnung.

Ein solcher Deckel kann vor der Heißdampfsterilisierung am Fluidanschluss der Separationseinheit angebracht werden und bis zum Zusammenbau der Einweg-Vorrichtung dort verbleiben. Der besondere Verschlussmechanismus erlaubt es, dass der Fluidanschluss wahlweise mit der Umgebung in Strömungsverbindung gebracht oder von der Umgebung getrennt werden kann (zumindest um einem unerwünschten Partikeleintrag entgegenzuwirken), ohne dass der Deckel für das Freigeben und Verschließen vom Fluidanschluss abgenommen werden muss. Wie oben bereits erläutert, ist beim erfindungsgemäßen Verfahren vorgesehen, dass während der Sterilisierung mit Heißdampf die Durchgangsöffnung freigegeben ist, um einen Druckausgleich zu ermöglichen. Danach wird die Durchgangsöffnung verschlossen, um die gewünschte Partikelbarriere gegenüber der Umgebung zu bilden.

Gemäß einer bevorzugten Konstruktion des erfindungsgemäßen Deckels weist der Mechanismus einen Schließkörper auf, der zwischen einer Verschlussstellung, in der der Schließkörper die wenigstens eine Durchgangsöffnung abdeckt, und einer Öffnungsstellung überführbar ist, in der der Schließkörper die wenigstens eine Durchgangsöffnung nicht abdeckt. Das Abdecken und Freigeben erfolgt also nicht durch das Anbringen und Abnehmen des Deckels, sondern jeweils bei angebrachtem Deckel durch eine Bewegung des Schließkörpers.

Der Mechanismus zum Verschließen und Freigeben der Durchgangsöffnung sollte auch dann manuell betätigbar sein, wenn sich die Separationseinheit in der geschlossenen Hülle befindet. Hierfür weist der Deckel ein Betätigungselement auf, das leicht ertastbar und greifbar ist.

Im Gegensatz zur Separationseinheit selbst kann der Deckel eine wiederverwendbare Komponente sein, die vorzugsweise zumindest überwiegend aus Edelstahl gebildet ist. Wenn der Deckel nach dem Aufbau der Einweg-Vorrichtung nicht mehr benötigt wird, kann er nach entsprechender Reinigung für den gleichen Zweck bei einer anderen Einweg-Vorrichtung eingesetzt werden.

Weitere Merkmale und Vorteile der Erfindung ergeben sich aus der nachfolgenden Beschreibung und aus den beigefügten Zeichnungen, auf die Bezug genommen wird. In den Zeichnungen zeigen:
- Figur 1 eine perspektivische Ansicht einer Einweg-Vorrichtung zur Durchführung eines bioverfahrenstechnischen Prozesses;
- Figur 2 die Einweg-Vorrichtung aus Figur 1 in einer Draufsicht;
- Figur 3 eine Separationseinheit mit Anschlussschlauchleitungen und Konnektoren;
- Figur 4 einen Deckel für einen Fluidanschluss einer Separationseinheit;
- Figur 5 eine seitliche Explosionsansicht eines Verbindungsrohrs mit mehreren Fluidanschlüssen; und
- Figur 6 eine Draufsicht auf das Verbindungsrohr aus Figur 5.

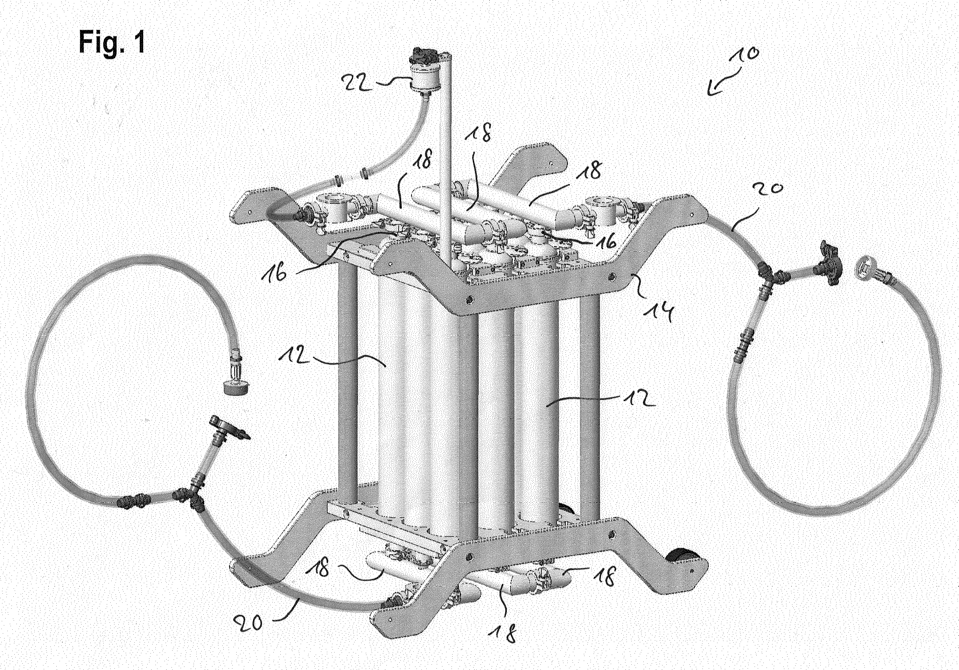

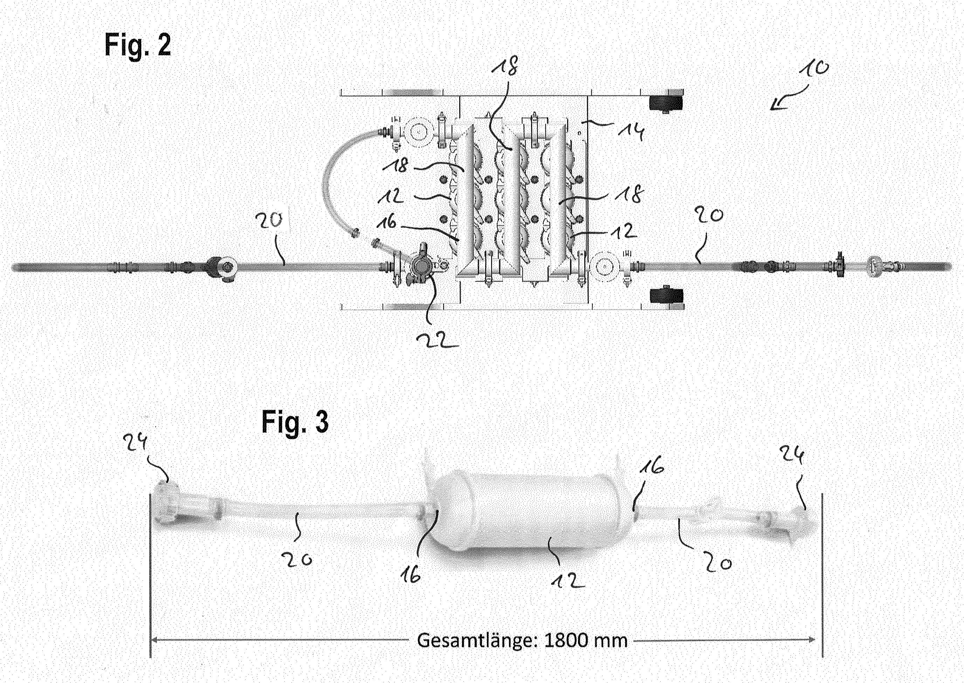

In den Figuren 1 und 2 ist eine Einweg-Vorrichtung 10 zur Durchführung eines bioverfahrenstechnischen Prozesses im zusammengebauten Zustand gezeigt. Die Einweg-Vorrichtung 10 umfasst mehrere Separationseinheiten 12, die in einer starren Halterung 14 in einem vorgegebenen Raster angeordnet sind. Bei den Separationseinheiten 12 kann es sich grundsätzlich um Filtercapsulen, Chromatographiesäulen oder Membranadsorber handeln.

Die Fluidanschlüsse (Ein- und Ausgänge) 16 der Separationseinheiten 12 sind über starre Verbindungsrohre 18 miteinander verschaltet. An die Verbindungsrohre 18 sind zum Zuführen und Abführen von Medium Anschlussschlauchleitungen 20 angeschlossen.

Außerdem ist an ein oberes Verbindungsrohr 18 ein Steril-Luftfilter 22 zum gemeinsamen Entlüften aller Separationseinheiten 12 angeschlossen.

Die Einweg-Vorrichtung 10 kann noch weitere Komponenten aufweisen. Der genaue Aufbau und die Betriebsweise der Einweg-Vorrichtung 10 sind für die hier beschriebene Erfindung jedoch nicht von wesentlicher Bedeutung.

Bei der Einweg-Vorrichtung 10 handelt es sich um eine "große" Vorrichtung für den Einsatz in der Produktion mit einem großen Mediumdurchsatz (Produktionsmaßstab). Davon abzugrenzen sind kleinere Vorrichtungen, die in Labors zur Entwicklung von Prozessen verwendet werden (Labormaßstab).

Die Einweg-Vorrichtung 10 soll als Ganzes und, soweit möglich, in sterilem Zustand oder zumindest mit einer möglichst geringen Keimbelastung (Bioburden) und sonstigen Verunreinigungen durch Partikel o.ä. an den Anwender ausgeliefert werden. Grundsätzlich ist in diesem Zusammenhang eine Sterilisierung durch Gammastrahlung bevorzugt, da bei diesem Verfahren in der Regel die Maßhaltigkeit der Komponenten gewährleistet werden kann.

Die Einweg-Vorrichtung 10 enthält im vorliegenden Fall jedoch wenigstens eine Separationseinheit 12, die nicht für eine Sterilisierung durch Gammastrahlung geeignet ist. Dies ist insbesondere dann der Fall, wenn das Filter- bzw. das Membranmaterial durch die bei einer Gammasterilisierung typischerweise verabreichte Energiedosis (z. B. 25 kGy) so stark beschädigt würde, dass eine bestimmungsgemäße Funktion nicht mehr garantiert werden kann. Beispiele für solche ungeeigneten Materialien sind Polytetrafluorethylen (PTFE), Polypropylen (PP) und Polyvinylchlorid (PVC).

Die nicht durch Gammastrahlung sterilisierbaren Separationseinheiten 12 werden als Untereinheiten einzeln oder in einem Verbund, ggf. mit weiteren Komponenten, mit Heißdampf in einem Autoklav sterilisiert. Eine solche Untereinheit darf nur so groß sein, dass sie im Autoklav untergebracht werden kann.

Bei der Einteilung der Einweg-Vorrichtung 10 in Untereinheiten ist neben den äußeren Abmessungen noch ein weiteres Kriterium zu beachten: Es muss sichergestellt sein, dass bei der Behandlung im Autoklav vor allem die inneren Flächen der Separationseinheiten 12 in ausreichendem Maße und lange genug mit dem Heißdampf in Berührung kommen. Dies ist insbesondere dann von Bedeutung, wenn mehrere Separationseinheiten 12 miteinander verbunden sind, wie etwa durch die Verbindungsrohre 18, oder an Schlauchleitungen 20 angeschlossen sind, sodass der Heißdampf nicht direkt durch die Fluidanschlüsse 16 in die Separationseinheiten 12 eindringen kann. Es besteht dann grundsätzlich die Gefahr, dass der Heißdampf nicht alle relevanten Flächen erreicht bzw. nicht schnell genug erreicht, um eine ausreichende Sterilisierung zu gewährleisten.

In der Praxis hat sich gezeigt, dass die vom Heißdampf zu durchströmende Gesamtlänge einer Untereinheit 1800 mm nicht überschreiten sollte. Dies ist in Figur 3 anhand eines Beispiels gezeigt, bei dem die zu durchströmende Gesamtlänge einer Filtercapsule 12 mit zwei angeschlossenen Schlauchleitungen 20 und Konnektoren 24 an den freien Enden etwa 1800 mm beträgt.

Nachfolgend wird anhand eines bevorzugten Ausführungsbeispiels beschrieben, wie eine vorgegebene Reinheit, insbesondere eine vorgegebene mikrobiologische Reinheit, der Einweg-Vorrichtung 10 bis zur Inbetriebnahme der gesamten Einweg-Vorrichtung 10 bei einem Anwender sichergestellt wird.

Zunächst wird die Heißdampfsterilisierung einer Untereinheit mit einer Separationseinheit 12 beschrieben. Diese Beschreibung ist ggf. auf die weiteren Separationseinheiten 12 derselben Untereinheit bzw. auf die Separationseinheiten 12 weiterer Untereinheiten zu übertragen.

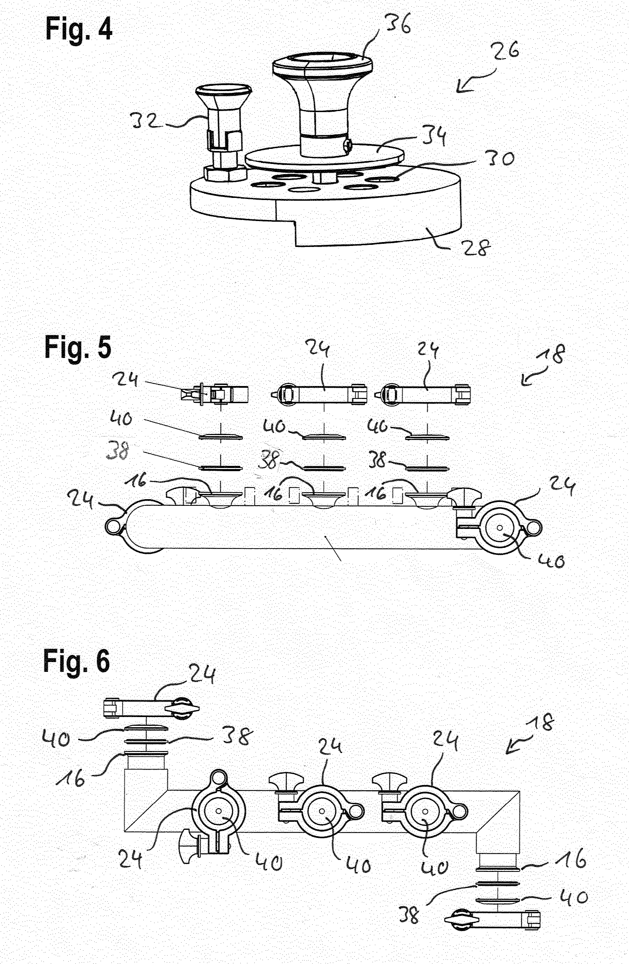

Diejenigen Stirnseiten der Separationseinheit 12, an denen sich ein oder mehrere freie Fluidanschlüsse 16 befinden (d. h. Fluidanschlüsse 16, an denen vor der Sterilisierung kein Verbindungsrohr 18 und keine Schlauchleitung 20 o.ä. angeschlossen ist), werden mit einem Verschluss versehen. Bei dem hier beschriebenen Ausführungsbeispiel kommt als Verschluss ein Deckel 26 zum Einsatz, wie er in Figur 4 gezeigt ist. Der überwiegend aus Edelstahl gebildete Deckel 26 ist eine Mehrweg-Komponente, d. h. er kann für weitere Sterilisierungsvorgänge verwendet werden.

Der Deckel 26 besteht im Wesentlichen aus einem Grundkörper 28 mit einer oder mehreren durchgehenden Durchgangsöffnungen 30, einem Arretierbolzen 32 und einem Verschlussmechanismus zum selektiven Freigeben und Verschließen der Durchgangsöffnungen 30.

Der Grundkörper 28 ist genau an die Außenkontur der zugeordneten Stirnseite der Separationseinheit 12 angepasst und kann mittels des Arretierbolzens 32 an der Separationseinheit 12 fixiert werden.

Der Verschlussmechanismus weist einen Schließkörper 34 auf, der zwei definierte, stabile Stellungen einnehmen kann: eine Verschlussstellung, in der der Schließkörper 34 fest am Grundkörper 28 anliegt und die Durchgangsöffnungen 30 abdeckt, und eine Öffnungsstellung, in der der Schließkörper 34 angehoben ist, sodass er die Durchgangsöffnungen 30 nicht abdeckt.

Der Verschlussmechanismus ist mittels eines Betätigungselements 36 manuell betätigbar, d. h. durch Drücken bzw. Ziehen am Betätigungselement 36 kann der Schließkörper 34 in die jeweils andere Stellung überführt werden.

In der fixierten Position des Deckels 26 stehen der bzw. die Fluidanschlüsse 16 der Separationseinheit 12 über die Durchgangsöffnungen 30 in Strömungsverbindung mit der unmittelbaren Umgebung der Separationseinheit 12, wenn sich der Schließkörper 34 in der Öffnungsstellung befindet. Befindet sich der Schließkörper 34 dagegen in der Verschlussstellung, besteht keine, bzw. so gut wie keine Strömungsverbindung zwischen dem Inneren der Separationseinheit 12 und der Umgebung, d h. ein möglicher Partikeleintrag ist weitgehend verhindert. Der Grundkörper 28 und der Schließkörper 34 sind so ausgelegt, dass der Deckel 26 in der Verschlussstellung ein Spaltmaß von höchstens 100 µm, vorzugsweise von höchstens 50 µm und weiter vorzugsweise von höchstens 10 µm, sodass entsprechend größere Partikel abgehalten werden.

Nach dem Anbringen des Deckels 26 auf der Stirnseite der Separationseinheit 12 (und ggf. eines weiteren Deckels 26 auf der anderen Stirnseite) wird die Untereinheit in eine Hülle gepackt, wobei vorher sichergestellt wird, dass die Durchgangsöffnungen 30 freigegeben sind. Die Hülle besteht aus Tyvek^{®} oder einem vergleichbaren Material, das einerseits wasserdicht ist und unter normalen Umgebungsbedingungen als Sterilbarriere dienen kann, andererseits aber durchlässig für Wasserdampf ist. Die Hülle wird durch Schweißen geschlossen.

Anschließend wird die Untereinheit in der Hülle in den Autoklav eingelegt und unter vorgegebenen Bedingungen mit Heißdampf sterilisiert (z. B. 40 min bei 121 °C oder 30 min bei 131 °C). Der Heißdampf gelangt durch die Hülle hindurch an die Außenflächen der Untereinheit und durch die freigegebenen Durchgangsöffnungen 30 in das Innere der Separationseinheit 12. Die Durchgangsöffnungen 30 sind hierfür ausreichend groß dimensioniert. Die Durchgangsöffnungen 30 sorgen während der Heißdampfsterilisierung auch dafür, dass ein ausreichender Druckausgleich stattfinden kann, sodass sich die Separationseinheit 12 nicht plastisch verformt.

Die Hülle bleibt nach der Sterilisierung geschlossen und dient bis zum Zusammenbau der Einweg-Vorrichtung 10 als Sterilbarriere für die Untereinheit.

Die übrigen Komponenten der Einweg-Vorrichtung 10 werden vor deren Zusammenbau durch Gammastrahlung sterilisiert. Vor der Bestrahlung werden die offenen Fluidanschlüsse 16 der Verbindungsrohre 18 und der Anschlussschlauchleitungen 20 ebenfalls mit Verschlüssen abgedeckt, hier in Form von Dichtungen 38 und Blindkappen 40, die mittels Konnektoren 24, wie etwa Tri-Clamp-Klemmverbindern, angebracht werden. Dies ist am Beispiel eines Z-förmigen Verbindungsrohrs 18 in den Figuren 5 und 6 gezeigt. Die Verschlüsse und etwaige Hilfsmittel zu deren Befestigung bestehen aus Gammastrahlen-resistentem Material und sind so ausgelegt, dass sie einen Partikeleintrag durch die Fluidanschlüsse 16 effektiv verhindern, wobei die Spaltmaße vergleichbar zu den bevorzugten Spaltmaßen bei dem durch den Deckel 26 abgedeckten Fluidanschluss 16 der Separationseinheit 12 sind. Grundsätzlich können die Verschlüsse auch als Sterilbarrieren ausgelegt sein.

Danach werden die Verbindungsrohre 18 und Anschlussschlauchleitungen 20 in eine Primärverpackung verpackt, die als Sterilbarriere dient. Die Sterilisierung dieser Komponenten erfolgt dann im verpackten Zustand, weshalb für die Primärverpackung ebenfalls ein Material zu wählen ist, dessen Eigenschaften durch Gammastrahlung in der für eine Sterilisierung üblichen Dosis nicht wesentlich beeinträchtigt werden.

Der Zusammenbau der Einweg-Vorrichtung 10 erfolgt entweder beim Hersteller oder - nach Transport der steril verpackten Untereinheiten und weiteren Komponenten - beim Anwender, und vorzugsweise auf einer Sicherheitswerkbank, die ein steriles Konnektieren ermöglicht, oder in einem durch Wände abgetrennten Bereich eines Reinraums.

Vor dem Auspacken der Untereinheit aus der Hülle werden die Durchgangsöffnungen 30 des Deckels 26 verschlossen. Dies geschieht bei geschlossener Hülle durch Ergreifen des Betätigungselements 36 und Betätigen des Verschlussmechanismus. Trotz der dazwischen befindlichen Hülle kann das markante Betätigungselement 36 durch Ertasten leicht ausfindig gemacht werden.

Durch das Verschließen der Durchgangsöffnungen 30 bildet der Deckel 26 auch nach dem Auspacken der Untereinheit einen Schutz vor Partikeleintrag, ggf. sogar eine Sterilbarriere, sodass ein Eindringen von Verunreinigungen in das Innere der Separationseinheit 12 weitgehend vermieden wird.

Ebenso dienen die Blindkappen 40 nach dem Auspacken als Schutz vor Partikeleintrag, bevorzugt sogar als Sterilbarrieren, und verhindern somit weitgehend, dass Verunreinigungen in das Innere der Verbindungsrohre 18 bzw. der Anschlussschlauchleitungen 20 eindringen können.

Dennoch sollte beim Zusammenbau der Einweg-Vorrichtung 10 die Hülle bzw. die Primärverpackung jeweils so spät wie möglich geöffnet werden, um die Exposition der mit den Verschlüssen abgedeckten Fluidanschlüsse 16 gegenüber der Umgebung so kurz wie möglich zu halten. Die Exposition kann aber dank der Verschlüsse insgesamt bis zu drei Stunden betragen. Bevorzugt ist jedoch weniger als zwei Stunden, noch besser weniger als eine Stunde und idealerweise weniger als eine halbe Stunde.

In dieser Zeit wird jede Separationseinheit 12 an ihren vorgesehenen Platz in der starren Halterung 14 gestellt. Die Verbindungsrohre 18 und Anschlussschlauchleitungen 20, soweit nicht bereits vormontiert, werden bereitgelegt. Erst unmittelbar vor dem Anschließen einer Komponente werden die Deckel 26 bzw. Blindkappen 40 von den für die jeweilige Verbindung benötigten Fluidanschlüssen 16 abgenommen. Nach dem Abnehmen eines Deckels 26 bzw. einer Blindkappe 40 sollte die Exposition des jeweiligen offenen Fluidanschlusses 16 gegenüber der Umgebung deutlich kürzer gehalten werden. Bevorzugt beträgt diese Zeitspanne weniger als zwei Minuten, besser weniger als eine Minute und idealerweise weniger als 30 Sekunden. Eine Montage in einer Reinraumklasse besser als ISO 8 ist unter diesen Voraussetzungen dann nicht zwingend erforderlich.

Üblicherweise werden auf die oben beschriebene Weise zunächst die Verbindungsrohre 18 an die Separationseinheiten 12 angeschlossen, bevor dann die Anschlussschlauchleitungen 20 an die Verbindungsrohre 18 angeschlossen werden. In jedem Fall ist am Ende ein in sich geschlossenes System hergestellt.

Sofern der Zusammenbau bereits beim Hersteller, also nicht beim späteren Anwender, erfolgt, wird die zusammengebaute Einweg-Vorrichtung 10 als Ganzes in eine als Sterilbarriere dienende Verpackung verpackt und an den Anwender ausgeliefert.

Nach dem Aufstellen bzw. Zusammenbau der Einweg-Vorrichtung 10 beim Anwender verbleiben die Blindkappen 40 an den freien Schlauchenden solange dort, bis die Anschlussschlauchleitungen 20 im Rahmen der Inbetriebnahme an eine Mediumzufuhr bzw. -abfuhr angeschlossen werden.

Als weitere Sicherheitsmaßnahmen sind für die oben beschriebenen Arbeitsschritte beim Zusammenbau der Einweg-Vorrichtung 10 das Anlegen steriler Handschuhe und eines Mundschutzes vorgesehen. Die ansonsten übliche Reinraumkleidung ist obligatorisch.

Für den Zusammenbau der Einweg-Vorrichtung 10 sind zwei Personen vorgesehen, insbesondere um die gewünschte kurze Exposition offener Fluidanschlüsse 16 zu erreichen. Die erste Person ist für das Auspacken und die Bereitstellung der Komponenten zuständig. Die zweite Person nimmt die Deckel 26 und die Blindkappen 40 von den Fluidanschlüssen 16 ab und stellt die vorgesehenen Strömungsverbindungen her, d. h. sie arbeitet vornehmlich an den Stellen, die kritisch für die Reinheit im Inneren der Medium-durchströmten Komponenten sind.

Zudem werden Arbeitsflächen, Werkezuge und Ablagebehälter gesondert gereinigt.

Die Einweg-Vorrichtung 10 mit deren Separationseinheiten und übrigen Komponenten wurde nur beispielhaft beschrieben. Zusätzlich oder alternativ zu den Separationseinheiten 12 können auch andere Funktionseinheiten vorgesehen sein, die nicht für eine Sterilisierung durch Gammastrahlung geeignet sind, wie etwa Sensoranordnungen mit elektronischen Datenspeichern, die durch Gammastrahlung beeinträchtigt würden.

Anstelle des beschriebenen Deckels 26 kann für die Separationseinheiten 16 bzw. anderen Funktionseinheiten auch ein anderer Verschluss mit vergleichbarer Funktionalität verwendet werden. Ein Beispiel hierfür ist ein Stopfen aus Silikon oder einem ähnlichen Material mit einem oder mehreren Schlitzen, der auf einem Fluidanschluss 16 einer Separationseinheit 12 angebracht wird. Beim Erhitzen im Autoklav dehnt sich der Stopfen aus, und die Schlitze bilden Durchgangsöffnungen, sodass Heißdampf durch die so gebildeten Durchgangsöffnungen in das Innere der Separationseinheit 12 gelangen kann. Beim anschließenden Abkühlen schrumpft der Stopfen wieder auf seine Normalgröße, sodass sich die Durchgangsöffnungen schließen und ein Eindringen von Verunreinigungen durch die Schlitze weitgehend vermieden wird. Es ist auch möglich, einen Luft- bzw. Heißdampf-durchlässigen Staubbeutel als Schutz um den Fluidanschluss 16 zu legen und dort zu fixieren.

Zum (selektiven) Verbinden der Separationseinheiten 12 bzw. Funktionseinheiten können auch mehrere direkt miteinander verbindbare, gammasterilisierbare Verteilerbaugruppen verwendet werden, die direkt an den Stirnseiten der Separationseinheiten 12 bzw. Funktionseinheiten angebracht werden.

### Bezuaszeichenliste

- 10: Einweg-Vorrichtung
- 12: Separationseinheit
- 14: Halterung
- 16: Fluidanschluss
- 18: Verbindungsrohr
- 20: (Anschluss-)Schlauchleitung
- 22: Steril-Luftfilter
- 24: Konnektor
- 26: Deckel
- 28: Grundkörper
- 30: Durchgangsöffnung
- 32: Arretierbolzen
- 34: Schließkörper
- 36: Betätigungselement
- 38: Dichtung
- 40: Blindkappe

## Patentansprüche

1. Verfahren zur Sicherstellung einer mikrobiologischen Reinheit einer Einweg-Vorrichtung (10) zur Durchführung eines bioverfahrenstechnischen Prozesses, wobei die Einweg-Vorrichtung (10) wenigstens eine gammasterilisierbare Komponente, die aus für eine Sterilisierung durch Gammastrahlung geeigneten Materialien gebildet ist, und wenigstens eine nicht-gammasterilisierbare Untereinheit umfasst, die ein für eine Sterilisierung durch Gammastrahlung ungeeignetes Material enthält, wobei sowohl die Komponente als auch die Untereinheit jeweils einen Bereich aufweisen, der bei der Durchführung des bioverfahrenstechnischen Prozesses mit einem Prozess-Medium in Berührung kommt, und wobei das Verfahren folgende Schritte umfasst:
a) Sterilisieren der gammasterilisierbaren Komponente durch Gammastrahlung;
b) Schützen des Medium-berührenden Bereichs der gammasterilisierbaren Komponente durch eine Sterilbarriere;
c) Sterilisieren der nicht-gammasterilisierbaren Untereinheit mit Heißdampf;
d) Schützen des Medium-berührenden Bereichs der nicht-gammasterilisierbaren Untereinheit durch eine Sterilbarriere;
e) Entfernen der Sterilbarrieren; und
f) Montieren der gammasterilisierten Komponente und der Heißdampf-sterilisierten Untereinheit in der Einweg-Vorrichtung (10) unmittelbar nach dem Entfernen der Sterilbarrieren.

2. Verfahren nach Anspruch 1, **dadurch gekennzeichnet, dass** der Zeitraum zwischen dem Entfernen einer Sterilbarriere und dem Montieren der zugehörigen gammasterilisierten Komponente bzw. der zugehörigen Heißdampf-sterilisierten Untereinheit jeweils kürzer als zwei Minuten, vorzugsweise kürzer als eine Minute und weiter vorzugsweise kürzer als eine halbe Minute ist.

3. Verfahren zur Sicherstellung einer mikrobiologischen Reinheit einer Einweg-Vorrichtung (10) zur Durchführung eines bioverfahrenstechnischen Prozesses, wobei die Einweg-Vorrichtung (10) wenigstens eine gammasterilisierbare Komponente, die aus für eine Sterilisierung durch Gammastrahlung geeigneten Materialien gebildet ist, und wenigstens eine nicht-gammasterilisierbare Untereinheit umfasst, die ein für eine Sterilisierung durch Gammastrahlung ungeeignetes Material enthält, wobei sowohl die Komponente als auch die Untereinheit jeweils einen Bereich aufweisen, der bei der Durchführung des bioverfahrenstechnischen Prozesses mit einem Prozess-Medium in Berührung kommt, und wobei das Verfahren folgende Schritte umfasst:
a) Sterilisieren der gammasterilisierbaren Komponente durch Gammastrahlung;
b) Schützen des Medium-berührenden Bereichs der gammasterilisierbaren Komponente durch eine Sterilbarriere;
c) Sterilisieren der nicht-gammasterilisierbaren Untereinheit mit Heißdampf;
d) Schützen des Medium-berührenden Bereichs der nicht-gammasterilisierbaren Untereinheit durch eine Sterilbarriere;
e) zusätzliches Abdecken des Medium-berührenden Bereichs der gammasterilisierbaren Komponente mit wenigstens einem ersten Verschluss, und/oder zusätzliches Abdecken des Medium-berührenden Bereichs der nicht-gammasterilisierbaren Untereinheit mit wenigstens einem zweiten Verschluss;
f) Entfernen der Sterilbarrieren und späteres Entfernen des ersten und/oder zweiten Verschlusses; und
g) Montieren der gammasterilisierten Komponente bzw. der Heißdampf-sterilisierten Untereinheit in der Einweg-Vorrichtung (10) unmittelbar nach dem Entfernen des ersten bzw. zweiten Verschlusses.

4. Verfahren nach Anspruch 3, **dadurch gekennzeichnet,**
**dass** der Zeitraum zwischen dem Entfernen der jeweiligen Sterilbarriere und dem Montieren der zugehörigen, mit einem ersten Verschluss versehenen gammasterilisierten Komponente bzw. der zugehörigen, mit einem zweiten Verschluss versehenen Heißdampf-sterilisierten Untereinheit jeweils kürzer als drei Stunden, vorzugsweise kürzer als zwei Stunden, weiter vorzugsweise kürzer als eine Stunde und weiter vorzugsweise kürzer als eine halbe Stunde ist, und
**dass** der Zeitraum zwischen dem Entfernen des ersten bzw. zweiten Verschlusses und dem Montieren der zugehörigen gammasterilisierten Komponente bzw. Heißdampf-sterilisierten Untereinheit jeweils kürzer als zwei Minuten, vorzugsweise kürzer als eine Minute und weiter vorzugsweise kürzer als eine halbe Minute ist.

5. Verfahren nach Anspruch 3 oder 4, **dadurch gekennzeichnet, dass** der erste und/oder der zweite Verschluss eine Partikeleintrag-reduzierende Barriere mit einem Spaltmaß von höchstens 100 µm, vorzugsweise von höchstens 50 µm, weiter vorzugsweise von höchstens 10 µm bildet.

6. Verfahren nach einem der vorhergehenden Ansprüche, **dadurch gekennzeichnet, dass** der Schritt b) vor dem Schritt a) und/oder der Schritt d) vor dem Schritt c) erfolgt.

7. Verfahren nach einem der vorhergehenden Ansprüche, **dadurch gekennzeichnet, dass** die Sterilbarriere für die Heißdampf-sterilisierte Untereinheit eine die Untereinheit vollständig umgebende, geschlossene Hülle umfasst.

8. Verfahren nach Anspruch 7, **dadurch gekennzeichnet, dass** die Hülle aus einem Vliesstoff, vorzugsweise Tyvek^{®}, gebildet ist.

9. Verfahren nach einem der vorhergehenden Ansprüche, **dadurch gekennzeichnet, dass** der Medium-berührende Bereich der Untereinheit vor dem Sterilisieren mit Heißdampf und bis zum Montieren der Heißdampf-sterilisierten Untereinheit durch einen zweiten Verschluss, insbesondere einen Deckel (26), abgedeckt ist, wobei der zweite Verschluss wenigstens eine Durchgangsöffnung (30) aufweist, die selektiv freigegeben und verschlossen werden kann.

10. Verfahren nach einem der vorhergehenden Ansprüche, **dadurch gekennzeichnet, dass** die nicht-gammasterilisierbare Untereinheit wenigstens eine Funktionseinheit, insbesondere eine Separationseinheit (12), mit einem Fluidanschluss (16) aufweist, der durch einen zweiten Verschluss abgedeckt wird.

11. Verfahren nach Anspruch 10, **gekennzeichnet durch** folgende Schritte bzw. Maßnahmen:
- Anbringen des zweiten Verschlusses auf dem Fluidanschluss (16) der Funktionseinheit;
- Freigeben der Durchgangsöffnung (30), falls die Durchgangsöffnung (30) verschlossen ist;
- Verpacken der nicht-gammasterilisierbaren Untereinheit in die Hülle;
- Sterilisieren der verpackten Untereinheit mit Heißdampf bei freigegebener Durchgangsöffnung (30);
- nach dem Sterilisieren mit Heißdampf: Verschließen der Durchgangsöffnung (30) in der geschlossenen Hülle;
- Auspacken der Heißdampf-sterilisierten Untereinheit aus der geschlossenen Hülle; und
- Abnehmen des zweiten Verschlusses unmittelbar vor dem Montieren der Untereinheit.

12. Verfahren nach einem der vorhergehenden Ansprüche, **dadurch gekennzeichnet, dass** der Medium-berührende Bereich der gammasterilisierbaren Komponente vor dem Sterilisieren durch Gammastrahlung und bis zum Montieren der gammasterilisierten Komponente durch einen ersten Verschluss, vorzugsweise durch eine Blindkappe (40) oder einen Sterilkonnektor, abgedeckt bleibt.

13. Verfahren nach Anspruch 12, **dadurch gekennzeichnet, dass** die gammasterilisierbare Komponente wenigstens ein Verbindungsrohr (18) und/oder eine Mehrzahl von untereinander verbindbaren Verteilerbaugruppen zum Verbinden mehrerer Separationseinheiten (12) und/oder wenigstens eine Anschlussschlauchleitung (20) umfasst, wobei die gammasterilisierbare Komponente wenigstens einen Fluidanschluss (16) aufweist, der durch den ersten Verschluss abgedeckt wird.

14. Verfahren nach einem der vorhergehenden Ansprüche, **dadurch gekennzeichnet, dass** die Sterilbarriere für die gammasterilisierbare Komponente eine die Komponente vollständig umgebende, geschlossene Primärverpackung umfasst.

15. Verfahren nach einem der vorhergehenden Ansprüche, **dadurch gekennzeichnet, dass** die Heißdampf-sterilisierte Untereinheit und die gammasterilisierte Komponente auf einer Sicherheitswerkbank oder in einem Reinraum, vorzugsweise in einem durch Wände abgetrennten Bereich eines Reinraums, montiert werden.

16. Deckel (26) für einen Fluidanschluss (16) einer Separationseinheit (12) einer nicht-gammasterilisierbaren Untereinheit zur Verwendung in einem Verfahren nach einem der Ansprüche 3 bis 15, wobei der Deckel (26) Folgendes umfasst:
- einen Grundkörper (28) zum passgenauen Anbringen des Deckels (26) am Fluidanschluss (16);
- wenigstens eine Durchgangsöffnung (30), die nach dem Anbringen des Deckels (26) am Fluidanschluss (16) eine Strömungsverbindung zwischen dem Medium-berührenden Bereich der Separationseinheit (12) und der unmittelbaren Umgebung der Separationseinheit (12) bereitstellt; und
- einen Mechanismus zum selektiven Freigeben und Verschließen der wenigstens einen Durchgangsöffnung (30), wobei der Mechanismus vorzugsweise einen Schließkörper (34) aufweist, der zwischen einer Verschlussstellung, in der der Schließkörper (34) die wenigstens eine Durchgangsöffnung (30) abdeckt, und einer Öffnungsstellung überführbar ist, in der der Schließkörper (34) die wenigstens eine Durchgangsöffnung (30) nicht abdeckt; und
- vorzugsweise ein Betätigungselement (36), mit dem der Mechanismus manuell betätigbar ist;
wobei der Deckel (26) vorzugsweise eine wiederverwendbare Komponente ist, die weiter vorzugsweise zumindest überwiegend aus Edelstahl gebildet ist.

17. Verwendung eines Deckels (26) nach Anspruch 16 in einem Verfahren nach einem der Ansprüche 3 bis 15.
